Europäisches Patentamt

European Patent Office

Office européen des brevets

(19)

(11) **EP 1 059 364 A1**

(12) ## DEMANDE DE BREVET EUROPEEN

(43) Date de publication:
**13.12.2000 Bulletin 2000/50**

(51) Int Cl.$^7$: **C23C 8/24**

(21) Numéro de dépôt: **99440137.0**

(22) Date de dépôt: **08.06.1999**

(84) Etats contractants désignés:
**AT BE CH CY DE DK ES FI FR GB GR IE IT LI LU MC NL PT SE**
Etats d'extension désignés:
**AL LT LV MK RO SI**

(71) Demandeur: **Crevoiserat, Jean-Michel**
**2944 Bonfol (CH)**

(72) Inventeur: **Crevoiserat, Jean-Michel**
**2944 Bonfol (CH)**

(74) Mandataire: **Nithardt, Roland**
**CABINET NITHARDT ET ASSOCIES**
**14 Boulevard Alfred Wallach**
**BP 1445**
**68071 Mulhouse Cédex (FR)**

(54) **Sonde pour réguler l'atmosphère gazeuse dans les processus de nitruration ou de nitrocarburation dans les fours à atmosphère ou basse pression**

(57) La présente invention concerne une sonde pour réguler l'atmosphère gazeuse en permettant de calculer avec précision le potentiel de nitruration Kn pour tous les types d'atmosphère de nitruration ou de nitrocarburation, sans être influencée par l'urée et qui permet dans les cas de nitrocarburation de réguler l'activité de carbone.

La sonde selon l'invention comprend, en circuit fermé, un module de raccordement (3) de la sonde (1) au four (2), un premier module (10) de mesure de l'hydrogène résiduel du gaz prélevé dans le four, un module de craquage (11) de l'ammoniac résiduel du gaz prélevé dans le four, un second module (12) de mesure de l'hydrogène mesurant la totalité de l'hydrogène résiduel, un module de régulation du débit (13) et un module d'aspiration (14) par effet Venturi du gaz prélevé dans le four (2). La sonde permet, en mesurant deux fois la quantité d'hydrogène, de calculer la quantité d'ammoniac résiduel, et donc le potentiel de nitruration Kn de manière reproductible. et ce pour toutes les atmosphères de nitruration et de nitrocarburation.

FIG. UNIQUE

EP 1 059 364 A1

**Description**

La présente invention concerne une sonde pour réguler l'atmosphère gazeuse dans les processus de nitruration ou de nitrocarburation dans les fours à atmosphère ou basse pression.

Au cours de la nitruration et de la nitrocarburation, deux couches se forment à la surface de l'acier à traiter: la couche de combinaison et la couche de diffusion ou couche blanche.

A l'heure actuelle, il n'existe aucun moyen pour contrôler la formation des nitrures dans l'acier et pour calculer véritablement les épaisseurs des couches.

C'est pourquoi, on utilise aujourd'hui des méthodes empiriques basées sur la mesure de l'atmosphère de nitruration ou de nitrocarburation. Avec une certaine composition de gaz utilisés pendant le traitement, et en fonction de divers paramètres, tels que le type d'acier, la nature de la pièce ou la température, on obtient des recettes pour reproduire des couches de nitruration de même nature et de même épaisseur pour les mêmes paramètres. Ce procédé nécessite en conséquence un contrôle rigoureux de l'atmosphère de nitruration pour assurer l'épaisseur des couches et certaines caractéristiques exigées sur ces couches. L'avantage de la régulation de l'atmosphère est la reproductibilité des couches sur un type d'acier donné, indépendamment de la charge.

En nitruration, l'atmosphère, constituée d'ammoniac $NH_3$ et d'azote $N_2$ ou de $NH_3$ et de $NH_3$ craqué, peut être contrôlée au moyen d'analyseurs de gaz, un analyseur $NH_3$ mesurant l'ammoniac résiduel et un analyseur $H_2$ mesurant l'hydrogène résiduel. En connaissant la quantité résiduelle de $NH_3$ et de $H_2$, on peut calculer le potentiel de nitruration "Kn". Les analyseurs étant extrêmement onéreux, on utilise généralement un seul analyseur $NH_3$ et la quantité résiduelle de $H_2$ est déterminée par calcul.

En nitrocarburation, le contrôle de l'atmosphère dépend des gaz utilisés qui sont très variables. Le problème est que lorsqu'on injecte de l'oxygène avec de l'ammoniac dans un four, il se forme de l'urée lors du prélèvement du gaz, ce qui ne permet pas l'utilisation normale d'un analyseur. En plus des analyseurs, il faut généralement ajouter un ou plusieurs régulateurs ainsi qu'un calculateur pour piloter les différents processus, d'où la mise en oeuvre d'une installation coûteuse.

La présente invention vise à pallier ces inconvénients en proposant une sonde pour réguler l'atmosphère gazeuse en permettant de calculer le potentiel de nitruration Kn pour tous les types d'atmosphère de nitruration ou de nitrocarburation, sans être influencée par l'urée et qui permet dans les cas de nitrocarburation de réguler l'activité de carbone.

Dans ce but, l'invention concerne une sonde du genre indiqué en préambule, caractérisée en ce qu'elle comprend un module de raccordement de la sonde au four, un premier module de mesure de l'hydrogène résiduel du gaz prélevé dans le four, un module de craquage de l'ammoniac résiduel du gaz prélevé dans le four, un second module de mesure de l'hydrogène mesurant la totalité de l'hydrogène résiduel, un module de régulation du débit et un module d'aspiration du gaz prélevé dans le four.

De préférence, les différents modules sont agencés de sorte que la sonde mesure l'atmosphère en circuit fermé.

D'une manière avantageuse, le module de raccordement comprend un tube de prélèvement reliant le four au premier module de mesure de l'hydrogène résiduel et un conduit de retour reliant le module de régulation du débit au four. Il peut être prévu, respectivement sur le tube de prélèvement et sur le conduit de retour, une vanne pour la fermeture dudit tube et dudit conduit. Lesdites vannes peuvent être actionnées manuellement ou par des unités de commande électriques.

Pour permettre la calibration des modules de mesure, il peut être prévu sur le tube de prélèvement une entrée pour l'injection de gaz.

D'une manière avantageuse, la sonde peut comprendre, dans le cas d'une nitrocarburation avec un gaz oxydant, un module de mesure de la pression partielle d'oxygène dans le gaz prélevé. De préférence, ledit module de mesure de la pression partielle d'oxygène comprend une sonde Lambda.

D'une manière avantageuse, le module de régulation du débit du gaz prélevé dans le four comprend une vanne à pointeau. Il peut être également prévu un contrôleur de débit massique dans le tube de prélèvement.

De préférence, le module d'aspiration du gaz prélevé dans le four comprend un conduit d'aspiration agencé pour déboucher dans le conduit de retour de manière à réaliser une aspiration par effet Venturi et une entrée pour l'injection d'azote et de gaz pour la calibration, ledit azote injecté étant de préférence chauffé à environ 400°C.

Avantageusement, ledit conduit d'aspiration est placé à proximité du module de craquage.

Il peut être prévu, en cas de nitruration basse pression, une pompe sur le conduit de retour du module de raccordement.

De préférence, la sonde selon l'invention comprend des moyens de chauffage et de régulation de la température des différents modules à une température supérieure à 132°C.

D'une manière avantageuse, la sonde selon l'invention est reliée à un calculateur agencé pour calculer le potentiel de nitruration Kn.

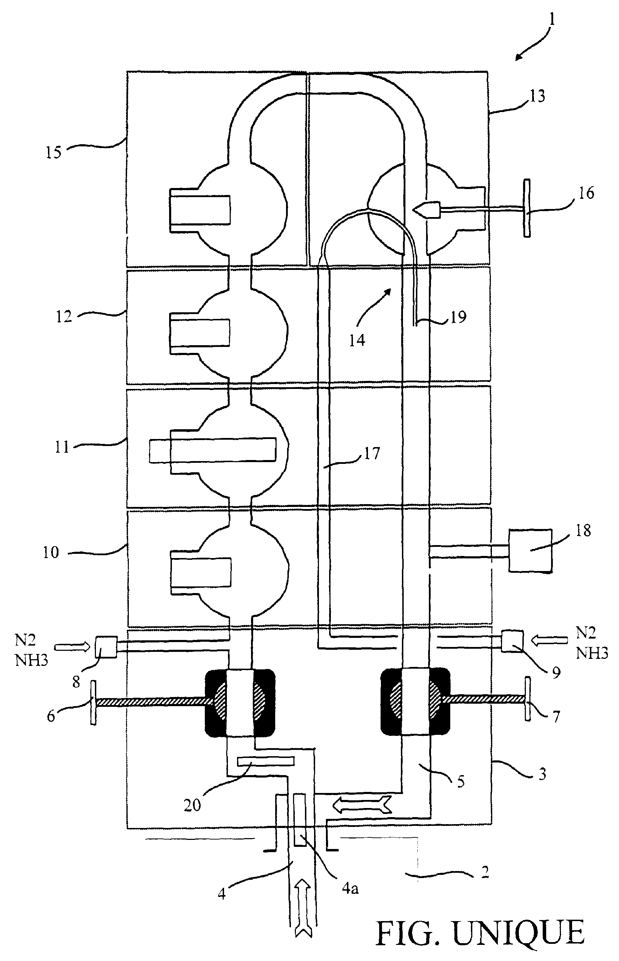

La présente invention et ses avantages apparaîtront mieux dans la description suivante d'un exemple de réalisation, en référence aux dessins annexés, dans lesquels la figure unique représente un schéma de principe de la sonde selon l'invention.

En référence à la figure unique, la sonde 1 selon l'invention est connectée à un four de nitruration ou de nitrocarburation 2 grâce à un module de raccordement 3. Elle comprend ensuite un premier module 10 de mesure de l'hydrogène résiduel du gaz prélevé dans le four 2, un module de craquage 11 de l'ammoniac résiduel du gaz prélevé dans le four 2, un second module 12 de mesure de l'hydrogène mesurant la totalité d'hydrogène résiduel, un module de régulation du débit 13 et un module d'aspiration 14 du gaz prélevé dans le four 2. Ces différents modules sont agencés en circuit fermé, c'est-à-dire qu'ils sont reliés les uns aux autres, le module de raccordement 3 étant lui même relié avec d'une part le premier module 10 de mesure d'hydrogène et avec d'autre part le module de régulation du débit 13.

Le module de raccordement 3 comprend un raccord du type Balzers®, un tube de prélèvement de gaz 4 en Inconel®, un réchauffage de canne 4a et un conduit de retour 5. Le tube de prélèvement 4 est relié au premier module 10 de mesure d'hydrogène tandis que le conduit de retour 5 est relié au module de régulation du débit 13 pour assurer le circuit fermé et pour réintroduire les gaz dans le four comme on le verra ci-après.

Sur le tube de raccordement 4 est prévue une vanne 6 permettant d'empêcher l'admission du gaz à analyser dans le premier module 10 de mesure d'hydrogène. Sur le conduit de retour 5 est prévue une vanne 7 permettant d'empêcher le retour des gaz provenant du module de régulation 13 dans le four 2. Les vannes 6 et 7 peuvent être actionnées manuellement ou être commandées par des unités de commande électriques (non représentées) pour permettre, comme on le verra ci-dessous, une calibration automatique de la sonde.

Entre la vanne 6 et le premier module 10 de mesure d'hydrogène est prévue une entrée 8 pour l'injection d'un gaz. Ce gaz peut être de l'azote $N_2$ pour calibrer le "zéro" des premier et second modules 10 et 12 de mesure d'hydrogène. Ce gaz peut également être de l'ammoniac $NH_3$ pour calibrer le "75%" d'hydrogène du second module 12 de mesure d'hydrogène.

Les modules 10 et 12 de mesure d'hydrogène sont équipés d'un capteur d'hydrogène $H_2$. En fonction de leur position dans le circuit, le module 10 mesure l'hydrogène résiduel du gaz prélevé dans le four 2 et le module 12 mesure la totalité de l'hydrogène résiduel.

Le module de craquage 11 comprend un craqueur qui craque complètement l'ammoniac $NH_3$ résiduel du gaz prélevé dans le four 2. La réaction de craquage, qui transforme l'ammoniac $NH_3$ en azote $N_2$ et en hydrogène $H_2$, s'effectue à plus de 950°C. Il est prévu également un catalyseur de la réaction de craquage.

En cas de nitrocarburation, il est prévu, entre le second module 12 de mesure d'hydrogène et le module de régulation 13, un module de mesure 15 de la pression partielle d'oxygène dans le gaz prélevé. Ce module 15 comprend une sonde Lambda et est recommandé s'il y a un gaz additionnel oxydant tel que CO, $CO_2$, $N_2O$, air, etc. Il permet de calculer et réguler l'activité de carbone. Par contre, ce module 15 n'a pas d'utilité en cas de nitrocarburation au $CH_4$ ou $C_3H_8$. Le module 15 peut être placé entre d'autres modules que les modules 12 et 13.

Le module de régulation du débit 13 permet de régler le débit de gaz à analyser grâce à une vanne à pointeau 16. Il fonctionne avec le module d'aspiration 14 pour permettre l'aspiration du gaz dans le four 2 par effet Venturi. A cet effet, le module d'aspiration 14 comprend un conduit d'aspiration 17 terminé par une buse 19 et agencé pour déboucher dans le conduit de retour 5, ainsi qu'une entrée 9 pour l'injection d'azote $N_2$. Lors de l'injection d'azote par l'entrée 9, on crée une dépression ce qui a pour effet d'aspirer le gaz dans le four 2. Ce dispositif permet de supprimer l'utilisation d'une pompe rendue difficile dans un milieu à température élevée. L'azote injecté par l'entrée 9 est chauffé à environ 400°C pour éviter la formation d'urée. D'une manière avantageuse, le conduit d'aspiration 17 est chauffé en le plaçant à proximité du module de craquage 11 pour bénéficier de la chaleur dégagée par ce module 11.

L'entrée 9 est également utilisée pour injecter de l'ammoniac $NH_3$ lorsque la vanne 7 est fermée de manière à calibrer le "75%" d'hydrogène du premier module 10 de mesure d'hydrogène.

Il est possible de prévoir, en cas de nitruration basse pression, une pompe 18 agencée sur le conduit de retour 5.

Tous les modules de la sonde sont équipés de moyens de chauffage et de régulation de la température (non représentés) permettant de maintenir une température supérieure à 132°C pour éviter la formation d'urée.

La sonde selon l'invention fonctionne de la façon suivante: les modules 10 et 12 de mesure d'hydrogène sont tout d'abord calibrés une fois par semaine ou une fois par jour. Si l'on souhaite une plus grande précision dans les mesures, le calibrage doit être fait avant chaque traitement. Pour cela, la vanne 6 est fermée et de l'azote est injecté par l'entrée 8 pour calibrer le "zéro" des modules 10 et 12. La vanne 6 étant fermée, on calibre le "75%" d'hydrogène du module 12 en injectant de l'ammoniac $NH_3$ par l'entrée 8. On ouvre ensuite la vanne 6 et on ferme la vanne 7 pour calibrer le "75%" d'hydrogène du module 10 en injectant de l'ammoniac $NH_3$ par l'entrée 9.

Les vannes 6 et 7 sont ensuite ouvertes et on injecte de l'azote dans le module d'aspiration 14 par l'entrée 9. Par effet d'aspiration, le gaz est alors prélevé dans le four 2 par le tube de prélèvement 4. Le gaz passe dans le premier module 10 de mesure d'hydrogène qui détermine la quantité d'hydrogène résiduel $H_2$four dans l'atmosphère du four 2. Le gaz passe ensuite dans le module de craquage 11 dans lequel l'ammoniac résiduel est entièrement craqué (+/- 100 ppm). Le gaz passe alors dans le second module 12 de mesure d'hydrogène qui mesure la totalité d'hydrogène résiduel.

Ces mesures sont transmises à un calculateur (non représenté) auquel est reliée la sonde 1 pour déterminer avec précision le taux d'ammoniac résiduel selon la formule:

$$\%NH_3r = \frac{(2x\%H_2crac)- (2x\%H_2four)}{3 - (2x(\%H_2crac/100))}$$

où

$NH_3r$ est la quantité d'ammoniac résiduel
$H_2crac$ est la quantité totale d'hydrogène craqué mesurée par le module 12
$H_2four$ est la quantité d'hydrogène résiduel dans le four mesurée par le module 10

Connaissant alors $NH_3r$, on peut calculer le potentiel de nitruration Kn de façon reproductible. La détermination du Kn peut se faire pour tous les types d'atmosphère de nitruration ou de nitrocarburation car la sonde n'est pas influençable par l'urée.

Dans certains cas de nitrocarburation, le module 15 permet, comme on l'a vu ci-dessus, de mesurer la pression partielle d'oxygène dans le gaz prélevé et de calculer l'activité de carbone.

La sonde fonctionnant en circuit fermé, les gaz prélevés ainsi que l'azote sont directement réinjectés dans le four par le conduit de retour 5. Le gaz reste toujours à une température supérieure à 132°C et grâce au module de régulation du débit 13, la sonde est continuellement balayée par un débit d'azote constant. La vanne à pointeau 16 permet le réglage définitif du débit en fonction notamment de la pression de travail dans le four 2. On peut également prévoir dans le tube de prélèvement 4 un contrôleur de débit massique 20 et un filtre, disposés entre la vanne 6 et le réchauffage de canne 4a.

D'autre part, les commandes électriques des vannes permettent une sélection automatique des gaz au niveau des entrées 8 et 9, et donc une calibration automatique de la sonde 1.

On peut également motoriser la vanne à pointeau 16 pour réguler le débit du gaz mesuré.

La sonde 1 selon l'invention ne possède aucune pièce en mouvement et ne nécessite en conséquence aucun entretien.

Elle offre également l'avantage de pouvoir être adaptée au process et aux gaz utilisés en combinant les différents modules qui la composent.

La présente invention n'est pas limitée à l'exemple de réalisation décrit mais s'étend à toute modification et variante évidente pour un homme du métier.

## Revendications

1. Sonde (1) pour réguler l'atmosphère gazeuse dans les processus de nitruration ou de nitrocarburation dans les fours à atmosphère ou basse pression, caractérisée en ce qu'elle comprend un module de raccordement (3) de la sonde (1) au four (2), un premier module (10) de mesure de l'hydrogène résiduel du gaz prélevé dans le four, un module de craquage (11) de l'ammoniac résiduel du gaz prélevé dans le four, un second module (12) de mesure de l'hydrogène mesurant la totalité de l'hydrogène résiduel, un module de régulation du débit (13) et un module d'aspiration (14) du gaz prélevé dans le four (2).

2. Sonde selon la revendication 1, caractérisée en ce que les différents modules sont agencés de sorte que la sonde (1) mesure l'atmosphère du four en circuit fermé.

3. Sonde selon la revendication 2, caractérisée en ce que le module de raccordement (3) comprend un tube de prélèvement (4) reliant le four (2) au premier module (10) de mesure de l'hydrogène résiduel et un conduit de retour (5) reliant le module de régulation (13) du débit au four (2).

4. Sonde selon la revendication 3, caractérisée en ce que le module de raccordement (3) comprend, respectivement sur le tube de prélèvement (4) et sur le conduit de retour (5), une vanne (6, 7) pour la fermeture dudit tube (4) et dudit conduit (5).

5. Sonde selon la revendication 4, caractérisée en ce qu'il est prévu des unités de commande électriques pour lesdites vannes (6, 7).

6. Sonde selon la revendication 4, caractérisée en ce qu'il est prévu sur le tube de prélèvement (4) une entrée (8) pour l'injection de gaz pour la calibration.

7. Sonde selon la revendication 1, caractérisée en ce qu'elle comprend, dans le cas d'une nitrocarburation avec un gaz oxydant, un module (15) de mesure de la pression partielle d'oxygène dans le gaz prélevé.

8. Sonde selon la revendication 7, caractérisée en ce que ledit module (15) de mesure de la pression partielle d'oxygène comprend une sonde Lambda.

9. Sonde selon la revendication 1, caractérisée en ce que le module de régulation du débit (13) du gaz prélevé dans le four comprend une vanne à pointeau (16).

10. Sonde selon la revendication 3, caractérisée en ce qu'il est prévu un contrôleur de débit massique (20) dans le tube de prélèvement (4).

11. Sonde selon la revendication 3, caractérisée en ce que le module d'aspiration (14) du gaz prélevé dans le four comprend un conduit d'aspiration (17) agencé pour déboucher dans le conduit de retour (5) de manière à réaliser une aspiration par effet Venturi et une entrée (9) pour l'injection d'azote et de gaz pour la calibration.

**12.** Sonde selon la revendication 11, caractérisée en ce que l'azote injecté est chauffé à environ 400°C.

**13.** Sonde selon la revendication 11, caractérisée en ce que le conduit d'aspiration (17) est placé à proximité du module de craquage (11).

**14.** Sonde selon la revendication 3, caractérisée en ce qu'il est prévu, en cas de nitruration basse pression, une pompe (18) sur le conduit de retour (5) du module de raccordement (3).

**15.** Sonde selon l'une quelconque des revendications précédentes, caractérisée en ce qu'elle comprend des moyens de chauffage et de régulation de la température des différents modules (3, 10 à 15) à une température supérieure à 132°C.

**16.** Sonde selon la revendication 1, caractérisée en ce qu'elle est reliée à un calculateur agencé pour calculer le potentiel de nitruration Kn.

FIG. UNIQUE

**Office européen** **RAPPORT DE RECHERCHE EUROPEENNE**

**des brevets**

Numéro de la demande

EP 99 44 0137

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| Y | US 4 417 927 A (FULLMAN ROBERT L) 29 novembre 1983 (1983-11-29) * colonne 3, ligne 13 - colonne 8, ligne 31; figures * | 1 | C23C8/24 |
| Y | EP 0 021 208 A (AICHELIN GMBH) 7 janvier 1981 (1981-01-07) * page 5, ligne 1 - page 7, colonne 34; figures * | 1 | |
| A | EP 0 813 059 A (CREVOISERAT JEAN MICHEL) 17 décembre 1997 (1997-12-17) * colonne 6, ligne 32 - colonne 13, ligne 11; figures * | 1-16 | |
| A | DE 196 44 051 A (HARTUNG REINHOLD DR ;MOEBIUS HANS HEINRICH PROF DR (DE)) 7 mai 1998 (1998-05-07) * le document en entier * | 1-16 | |
| A | DE 39 35 486 A (THAELMANN SCHWERMASCHBAU VEB) 28 juin 1990 (1990-06-28) * page 2, ligne 36 - page 3, ligne 51; figures * | 1-16 | **DOMAINES TECHNIQUES RECHERCHES (Int.Cl.7)** <br><br> C23C |
| A | FR 2 725 015 A (INNOVATIQUE SA) 29 mars 1996 (1996-03-29) * page 4, ligne 19 - page 9, ligne 34; figures * | 1-16 | |
| A | EP 0 118 014 A (SIEMENS AG) 12 septembre 1984 (1984-09-12) * page 3, ligne 21 - page 6, ligne 16; figures * | 1-16 | |

-/--

Le présent rapport a été établi pour toutes les revendications

| Lieu de la recherche | Date d'achèvement de la recherche | Examinateur |
|---|---|---|
| LA HAYE | 16 décembre 1999 | Bosma, R |

CATEGORIE DES DOCUMENTS CITES

X : particulièrement pertinent à lui seul
Y : particulièrement pertinent en combinaison avec un autre document de la même catégorie
A : arrière–plan technologique
O : divulgation non–écrite
P : document intercalaire

T : théorie ou principe à la base de l'invention
E : document de brevet antérieur, mais publié à la date de dépôt ou après cette date
D : cité dans la demande
L : cité pour d'autres raisons

........................................................
& : membre de la même famille, document correspondant

EPO FORM 1503 03.82 (P04C02)

Office européen
des brevets

**RAPPORT DE RECHERCHE EUROPEENNE**

Numéro de la demande

EP 99 44 0137

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | DE 197 19 225 C (LEVERKUS VOLKER DIPL ING) 6 août 1998 (1998-08-06) * colonne 4, ligne 31 - colonne 7, ligne 63; figures * --- | 1-16 | |
| A | DE 196 52 125 C (LEVERKUS VOLKER DIPL ING) 30 avril 1998 (1998-04-30) * colonne 4, ligne 48 - colonne 9, ligne 27; figures * ----- | 1-16 | |

**DOMAINES TECHNIQUES RECHERCHES (Int.Cl.7)**

Le présent rapport a été établi pour toutes les revendications

| Lieu de la recherche | Date d'achèvement de la recherche | Examinateur |
|---|---|---|
| LA HAYE | 16 décembre 1999 | Bosma, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 99 44 0137

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-12-1999

| Document brevet cité au rapport de recherche | | Date de publication | Membre(s) de la famille de brevet(s) | | Date de publication |
|---|---|---|---|---|---|
| US 4417927 | A | 29-11-1983 | AUCUN | | |
| EP 0021208 | A | 07-01-1981 | DE | 2923285 A | 11-12-1980 |
| | | | AT | 3215 T | 15-05-1983 |
| | | | BR | 8008706 A | 28-04-1981 |
| | | | WO | 8002698 A | 11-12-1980 |
| | | | JP | 56501013 T | 23-07-1981 |
| EP 0813059 | A | 17-12-1997 | FR | 2749937 A | 19-12-1997 |
| DE 19644051 | A | 07-05-1998 | AUCUN | | |
| DE 3935486 | A | 28-06-1990 | DD | 279508 A | 06-06-1990 |
| FR 2725015 | A | 29-03-1996 | DE | 707661 T | 10-10-1996 |
| | | | EP | 0707661 A | 24-04-1996 |
| | | | WO | 9529269 A | 02-11-1995 |
| EP 0118014 | A | 12-09-1984 | DE | 3304244 A | 09-08-1984 |
| | | | JP | 59148852 A | 25-08-1984 |
| DE 19719225 | C | 06-08-1998 | AUCUN | | |
| DE 19652125 | C | 30-04-1998 | AUCUN | | |

EPO FORM P0460

Pour tout renseignement concernant cette annexe : voir Journal Officiel de l'Office européen des brevets, No.12/82